# EUROPEAN PATENT SPECIFICATION

(11) **EP 1 171 174 B1**
(45) Date of publication and mention of the grant of the patent: **17.11.2004**
(21) Application number: 99928274.2
(22) Date of filing: 16.04.1999
(51) Int. Cl.: A61L 27/06, C21D 11/00, A61F 2/02

(54) **SUPERFICIAL STRUCTURE ON SURFACE OF PROSTHETIC IMPLANTS MADE OF TITANIUM METAL OR TITANIUM BASED ALLOY**
OBERFLÄCHENSTRUKTUR AUF DER OBERFLÄCHE VON PROSTHETISCHEN IMPLANTATEN AUS TITAN ODER TITANLEGIERUNGEN
STRUCTURES SUPERFICIELLES DES SURFACES D'IMPLANTS PROTHETIQUES EN TITANE OU EN ALLIAGE A BASE DE TITANE

(43) Date of publication of application: 16.01.2002
(73) Proprietor: Nobel Biocare AB (publ), 402 26 Göteborg (SE)
(72) Inventor: PETO, Gábor, H-1121 Budapest (HU); KARACS, Albert, H-1121 Budapest (HU); PASZTI, Zoltán, H-1121 Budapest (HU); DIVINYI, Tamás, H-1221 Budapest (HU); FULLER, Ferenc, H-2310 Szigetszentmiklos (HU)
(74) Representative: Olsson, Gunnar
(86) International application number: PCT/SE1999/000603
(87) International publication number: WO 2000/062831

(56) References cited:
- EP-A1- 0 411 322
- EP-A1- 0 909 553
- EP-A2- 0 248 117
- WO-A1-90/14447
- US-A- 5 222 983
- DATABASE WPI Week 198832, Derwent Publications Ltd., London, GB; AN 1998-224913, XP002921557 'Treating Titanium (Alloy) Surface to Improve Bone Compatibility-by Laser Beam Irradiation to Form Hollows of Controlled Sized' & JP 63 160 662 A (KAWASAKI STEEL CORP.) 04 July 1988

## Description

The invention relates to the definition and production of superficial structures - that is the morphology and chemical composition of the surface - of element, screws, tooth-roots, that is of prosthetic implant to be fixed into bones, made of Ti metal or alloys of at least 98% Ti content, which structure considerably improves the integration of implants with bones and considerably decreases the number of occurring failures and complications.

The superficial structure of prosthetic implants is of crucial importance from the point of view of the successfulness of the implantation operation itself and of the long range (lasting for decades) behaviour of the implants. Thus for the production and for the structuring of the surface a great number of known procedures are applied.

Known procedure is the blasting of the implant surface with Al₂O₃ powder, glass bead, etc. and thus the roughening thereof. Although this method brings about a considerable increase of the surface area, it is easily seen however that some portion of the particles "shot" on the surface with a high pressure (energy) will get stuck into it in a self-locking manner and this may cause various types of complications. Our examination show that the superficial composition is influenced unfavourably with the application of this procedure and practically in an irreversible manner. The influence of the sharp acicular morphological elements on the process of integration into the bone cannot be considered definitely advantageous.

Known procedure is the treatment of the implant surface by forming a new layer with the deposition of additional material in such a way that the morphology of the surface is also modified towards obtaining larger surface area. The most common of such procedures is the so called plasma sputtering in which small grains of the layer material are melted and shot on the surface with high velocity. Various difficulties may arise in this case, e.g. it is hard to keep the shape and size constant, the layer material becomes of spongy structure, it may contain closed voids, as well as extraneous contaminant materials originated the abrasion of the so called sputtering tube, etc. In spite of all these disadvantages a considerable portion of the implants put on the market these days has got such type of surface structure.

Such procedures are known, in which a porous layer is produced by anodic oxidation of the titanium surface and then a chemical, electrochemical treatment of this layer is carried out (e.g. Japanese patent No. JP 5658990). In Hungarian patent number HU 213001B a ceramic oxide layer is produced by electrochemical, thermochemical treatment of the anodic oxide layer. In relation with these procedures the necessary application of different chemical materials causes a problem, since residual portions of them may remain in the porous structures. The same can be said about roughened surfaces produced possibly by simple chemical etching.

Such procedure is also known in which, in order to avoid difficulties in the shaping process of the surface morphology, smooth non-roughened, very carefully cleaned implants were applied with good results.

It is seen from the above that the morphological treatments resulting in large surface areas are usually accompanied by surface contaminations which may cause probable complications sooner or later, that is such treatment influence to some extent harmfully the inherent bioinert nature of titanium.

Thus it is concievable that there exists such procedure (European patent No. 0475358 A1) in which the final shaping of the morphology is carried out by using a "clean tool" that is laser light. According to this procedure the focused light of a XeCl laser drills crater-like sack-borings into the surface of the implant with 25-100 µm diameter, 40-60 µm depth and 30-50 holes/mm² surface density. In this case the laser treatment is applied definitely for the sake of shaping of the morphology and affects only the surface of the sack-holes.

The aim of our invention is to define and produce such a surface structure which, on one hand consists of appropriate macro-/and micromorphological elements, on the other hand ensures the presence of either the bulk composition or suitably modified chemical composition on the surface of the implant by "peeling off " the superficial layer possibly contaminated during previous treatments.

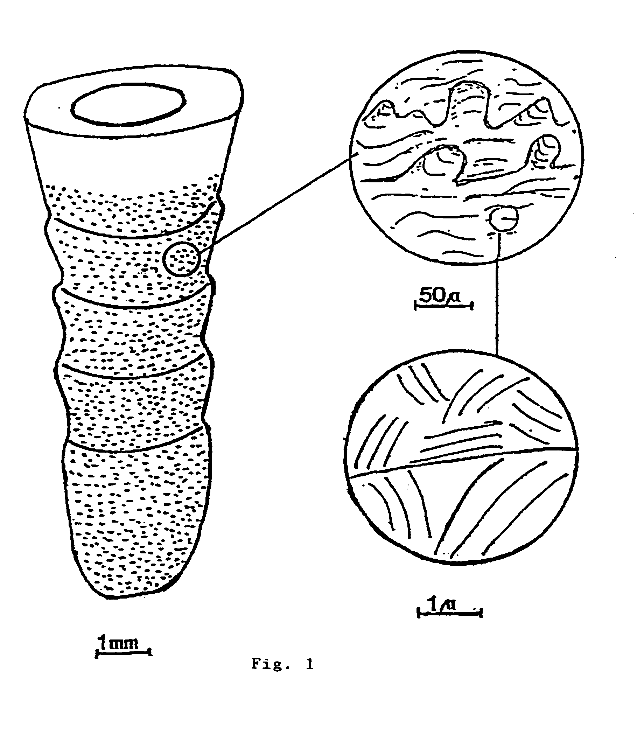

According to the example illustrated in fig 1. the surface treatment of a tooth-root implant - resulting in the superficial structure according to the invention - can be summarized as follows. The treatment is carried out by applying a short time radiation of great thermal effect - suitably laser pulse - either in a vacuum of better then 10⁻² Pa pressure or in a gas atmosphere of suitably selected composition and of pressure not greater then 100 Pa. The surface temperature will be higher then 5·10³ °C due to the high power density. The surface of the implant melts during the period of energy irradiation. The melted zone is of 40-50 µm depth. Due to the high temperature the superficial layer, including the possibly surface contaminations "explodes" down from the surface. The intact internal mass of the implant - which is large compared to the mass of the molten surface layer - cools down the temperature of the superficial layer below the melting point within several microseconds after terminating the irradiation. Resulting from the above mentioned physical processes the residual surface has got the following properties:
1* The surface of the melted superficial layer of 40-50 µm depth preserves the characteristic waveforms of the molten state in the form of regular and irregular morphological figures of 10-50 µm size.
2* The superfast cooling after the heating irradiation results in a superficial layer of fine-grain crystalline structure. Besides the morphological elements according to the above point 1* and superimposed thereupon there appear shapes of dendritic nature resembling to imprints of a acicular pine-needles usually in the size-domain of less than 1 µm.
3* Investigating the original and the treated surfaces by various methods of surface analysis one can observe the removal of the superficial contaminations.
4* The chemical composition of the molten zone can suitably be modified by various methods (e.g. gas atmosphere) in order to produce for example bioactive surface.
   The superficial structure according to the invention can be well distinguished from the other known structures and is favourable according to our investigations, since:
   1* The morphological figures of 10-50 µm size and of usually round-cornered shape can be jointed well to the size of the bone-cells. The bone-cells rapidly grow upon the structure izomorphic with the plane.
   2* The identically oriented surface elements of submicron morphological figures lend a good ground for the surface-protein interactions occurring in the nanometer size scale.
   3* The accomplishment of pure bioinert or bioactive surfaces reduces to minimum the probability of incidental complications.

## Claims

1. Superficial morphology of prosthetic implants made of titanium metal or titanium alloy consisting of partly regular partly irregular macromorphological elements of 10-50 µm size resembling to the rapidly frozen in surface of an intensely waving liquid as well as micromorphological elements of submicron size resembling to imprints of acicular pine-needles.

2. The superficial morphology according to claim 1), wherein said morphology is topologically izomorphic with the plane, and does not contain separated voids.

3. The morphology according to claim 1) and 2), wherein the treated surface of the implant is formed on the surface of the melted through and recrystallized material layer of 30-50 µm depth.

4. The surface according to claims 1-3), wherein surface does not contain alien contaminant materials different from the bulk composition.

5. The melted through surface layer according to claim 3), wherein the structure and/or the chemical composition of the layer are suitably modified and different from those of the bulk material.

6. The superficial structure according to claims 1-5), wherein the accomplishment of the structure is carried out suitably by using high-energy laser pulses either in vacuum or in a gas atmosphere of less the 100 Pa pressure containing suitably gaseous materials.

## Patentansprüche

1. Oberflächenstruktur von prosthetischen Implantaten aus Titanmetall oder Titanlegierung, bestehend aus teils regelmäßigen, teils unregelmäßigen makromorphologischen Elementen von 10-50 µm Größe, die der schnell eingefrorenen Oberfläche einer heftig wellenschlagenden Flüssigkeit ähneln, und aus mikromorphologischen Elementen mit einer Größe unter ein Mikron, die Abdrücken von acicularen Tannennadeln ähneln.

2. Oberflächenstruktur nach Anspruch 1, bei der die Struktur topologisch izomorph mit der Ebene ist und keine separaten Hohlräume enthält.

3. Struktur nach Anspruch 1 und 2, bei der die behandelte Oberfläche des Implantats auf der Oberfläche der durchgeschmolzenen und rekristallisierten Materialschicht von 30-50 µm Tiefe ausgebildet ist.

4. Oberfläche nach den Ansprüchen 1 bis 3, bei der die Oberfläche keine fremden kontaminierenden Materialien enthält, die von der Zusammensetzung des Grundmaterials verschieden sind.

5. Durchgeschmolzene Oberflächenschicht nach Anspruch 3, bei der die Struktur und/oder die chemische Zusammensetzung der Schicht in geeigneter Weise modifiziert und von der des Grundmaterials verschieden sind.

6. Oberflächenstruktur nach den Ansprüchen 1 bis 5, bei der das Zustandekommen der Struktur in geeigneter Weise durchgeführt wird durch Verwendung von hochenergetischen Laserimpulsen entweder im Vakuum oder in einer Gasatmosphäre von weniger als 100 Pa Druck, die geeignete gasförmige Materialien enthält.

## Revendications

1. Morphologie superficielle d'implants prothétiques constitués de titane métallique ou d'un alliage de titane consistant en des éléments macromorphologiques partiellement réguliers partiellement irréguliers de 10 à 50 µm de taille ressemblant à la surface rapidement congelée d'un liquide à ondulation intense de même que des éléments micromorphologiques de la taille du sous-micron ressemblant à des impressions d'aiguilles de pin aciculaires.

2. Morphologie superficielle selon la revendication 1, dans laquelle ladite morphologie est topologiquement isomorphe avec le plan, et ne contient pas de vides séparés.

3. Morphologie superficielle selon la revendication 1 et 2, dans laquelle la surface traitée de l'implant est formée sur la surface de la couche de matériau fondue et recristallisée de 30 à 50 µm de profondeur.

4. Surface selon les revendications 1 à 3, dans laquelle la surface ne contient pas de matériaux contaminants étrangers différents de la composition brute.

5. Couche fondue à travers la surface selon la revendication 3, dans laquelle la structure et/ou la composition chimique de la couche sont modifiées de manière appropriée et sont différentes de celles du matériau brut.

6. Structure superficielle selon les revendications 1 à 5, dans laquelle l'accomplissement de la structure est effectif de manière appropriée en utilisant des impulsions laser d'énergie élevée soit sous vide soit dans une atmosphère de gaz inférieure à 100 Pa de pression contenant de manière appropriée des matériaux gazeux.
